# EUROPEAN PATENT APPLICATION

(11) **EP 1 736 107 A1**
(43) Date of publication of application: **27.12.2006**
(21) Application number: 05425449.5
(22) Date of filing: 21.06.2005
(51) Int. Cl.: A61B 17/32, A61B 17/14, A61C 1/07, A61C 3/12

(54) **Tip for bone microsurgery**

(71) Applicant: Agabiti, Ivo, 61024 Monteciccardo (Pesaro) (IT)
(72) Inventor: Agabiti, Ivo, 61024 Monteciccardo (Pesaro) (IT)
(74) Representative: Modiano, Micaela Nadia

(57) **Abstract**

A tip for bone microsurgery, comprising at least one coupling (3), which is suitable for connection to an actuation handpiece, which is extended by at least one stem (4) ending with at least one head (5) adapted to produce cuts and/or incisions, which can be oriented in all the directions of space, in the portion of bone involved, the head (5) having, along at least one of the lateral sides, a distribution of sharp teeth (10) having a preset thickness, height and width.

## Description

The present invention relates to a tip for a bone microsurgery.

The particular field of bone microsurgery, with reference particularly but not exclusively to dental, maxillofacial, otorhinolaryngologic, neurological, orthopedic surgery and other kinds of surgery, uses extensively particular devices, such as piezoelectrically-operated handpieces, with which small bone cutting tips are associated.

As is known, these handpieces operate by virtue of the extremely rapid contractions of a quartz crystal subjected to an alternating current of suitable intensity, which produce the oscillation of the cutting tip at a frequency that can be as high as 30,000 Hz.

The cutting tips currently used in the field of surgery are generally constituted by a stem, which is appropriately curved in order to facilitate access to the parts to be treated and ends with a portion suitable to cut, produce incisions, or work the bone in the part involved. Known types of tip are generally shaped so as to be used essentially with their end: in other words, the portion that is sharp or serrated or otherwise suitable for cutting or incision is provided at the free end and is arranged substantially parallel to the longitudinal axis of symmetry of the stem of said bit.

This fact in itself reveals a first main drawback: these bits, in order to transmit such a rapid motion and at the same time be able to withstand fracture, must be sized appropriately, but currently they are never thinner than 0.55 mm, and this entails extreme difficulty in providing narrow and precise incisions.

In addition to this, there are other limitations of the described technique, which arise when using a piezoelectric handpiece, mentioned above, which is first of all capable of generating an oscillation of the tip only along a preset direction, which entails a continuous change of direction of said handpiece; secondly, the extremely high frequency at which the piezoelectric handpiece operates, together with the considerable dimensions of the friction surface, usually generates a large amount of heat, which must then be dissipated effectively by providing suitable cooling means in order to avoid damaging the bone tissue.

The aim of the present invention is to obviate the drawbacks described above by providing a tip for bone microsurgery that allows to perform narrow incisions, measuring 0.20 mm or less, which are very precise, by virtue of the better control determined by the reduced cutting frequency (subsonic 6000 Hz, ultrasonic 30,000 Hz) and for the same reasons produces a more modest temperature increase: the patient in fact appears to withstand better a sonic vibration rather than an ultrasonic vibration at 30,000 Hz.

Within this aim, an object of the present invention is to provide a tip for bone microsurgery that is versatile in use and is capable of performing simply interventions that are instead difficult to perform with conventional tips.

Another object of the present invention is to provide a tip for bone microsurgery that has a reduced thickness (approximately 0.20 mm) and is suitable to ensure maximum visibility in all operating conditions; since the tip according to the invention can also work with its sides, this determines a directionality of incision that is unlimited in the three spatial directions, facilitating the operator, who can produce cuts with a depth of even 10 mm or more.

A further object of the present invention is to provide a tip that is simple, relatively easy to provide in practice, safe in use, effective in operation, and relatively modest in cost.

This aim and these and other objects which will become better apparent hereinafter are achieved by the present tip for bone microsurgery, of the type that comprises at least one coupling, which is adapted for connection to an actuation handpiece, which is extended by at least one stem ending with at least one head adapted to produce cuts and/or incisions, which can be oriented in all the directions of space, in the portion of bone involved, characterized in that said head has, along at least one of the lateral sides, a distribution of sharp teeth having a preset thickness, height and width.

Further characteristics and advantages of the present invention will become better apparent and evident from the detailed description that follows of a preferred but not exclusive embodiment of a tip for bone microsurgery according to the invention, illustrated by way of non-limiting example in the accompanying drawings, wherein:

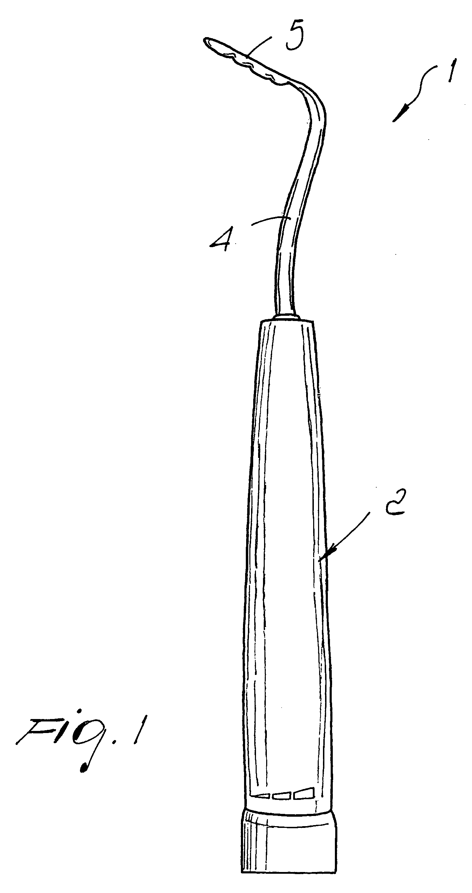

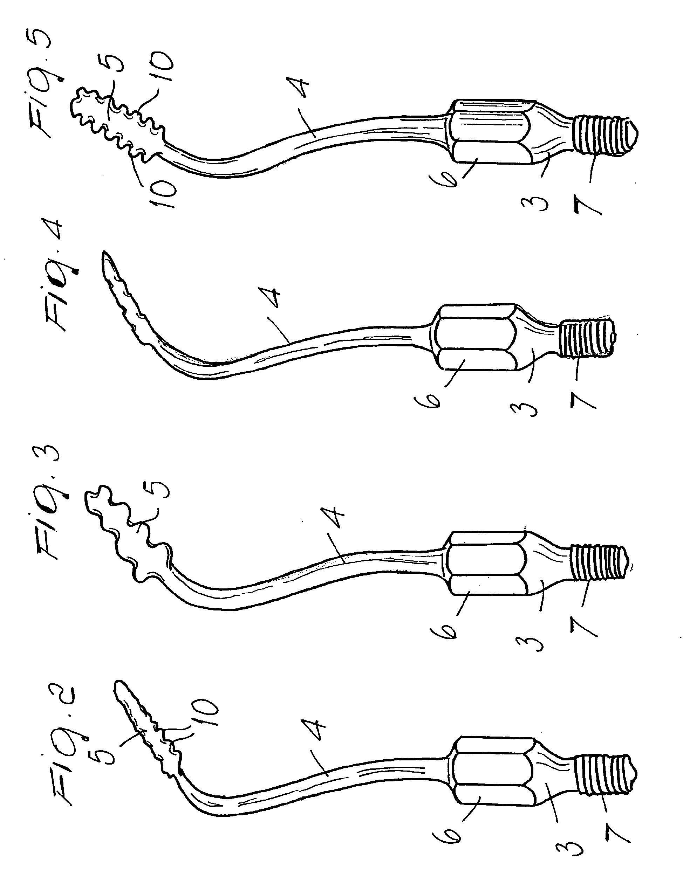

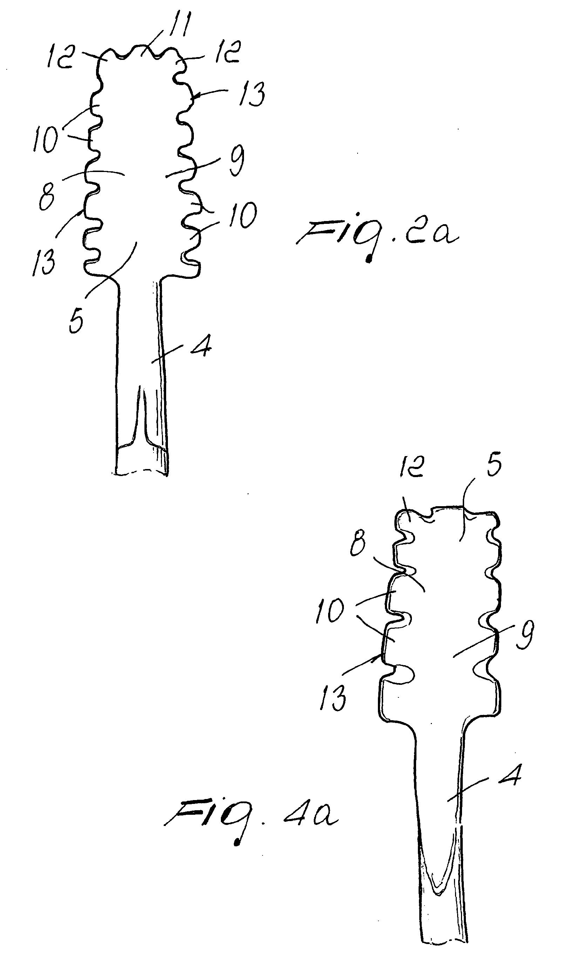

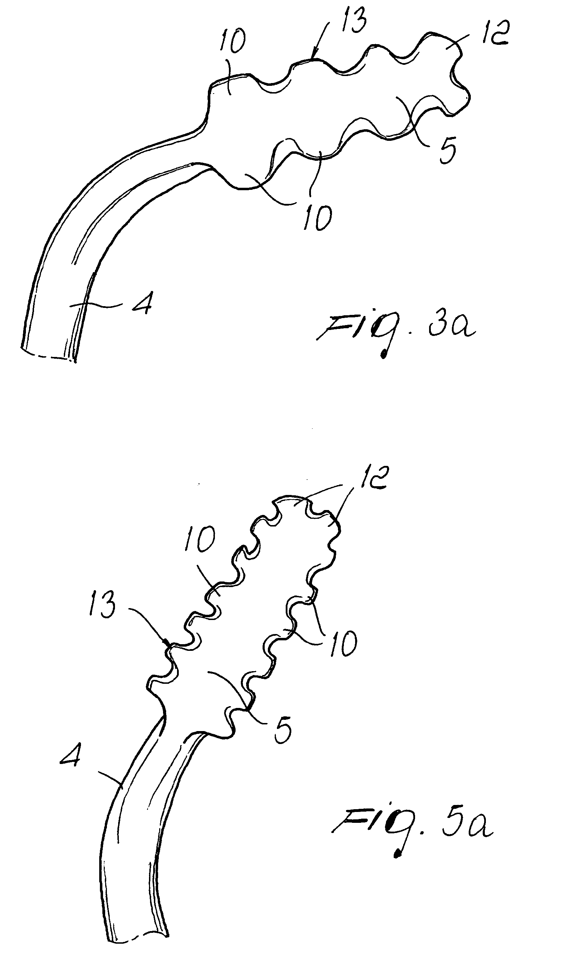

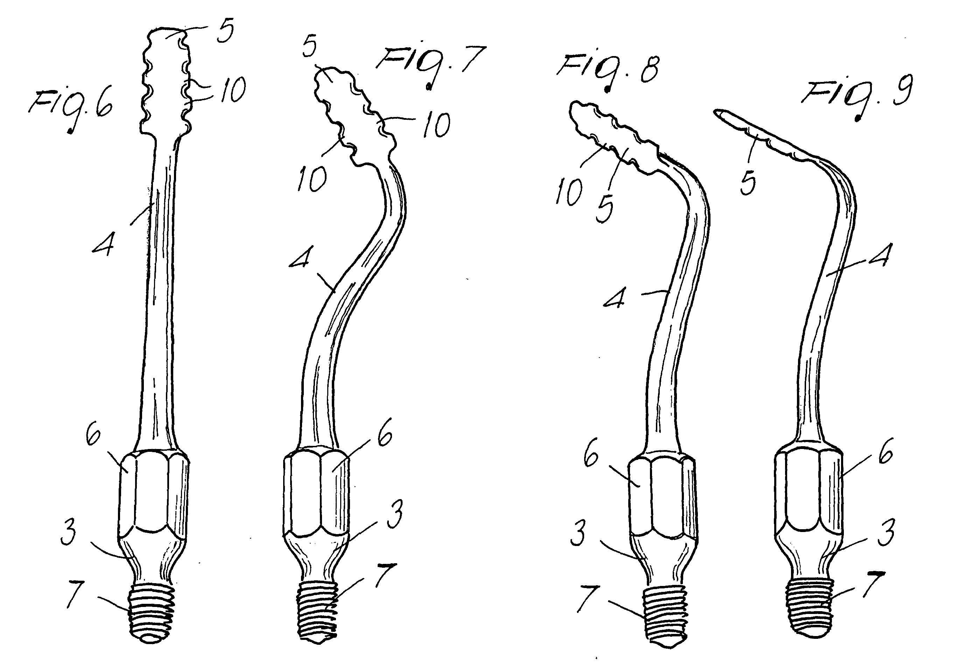

Figure 1 is a side view of a device for bone microsurgery, which comprises a tip according to the invention, in a first embodiment;
Figure 2 is a side view of the tip of Figure 1;
Figure 2a is a detail front view of the head of the tip of Figure 2;
Figure 3 is a side view of a second embodiment of the tip according to the invention;
Figure 3a is a view of a detail of Figure 3;
Figure 4 is a side view of a third embodiment of the tip according to the invention;
Figure 4a is a detail front view of the head of the tip of Figure 4;
Figure 5 is a front view of a fourth embodiment of the tip according to the invention;
Figure 5a is a view of a detail of Figure 5;
Figure 6 is a front view of a fifth embodiment of the tip according to the invention;
Figure 6a is a view of a detail of Figure 6;
Figure 7 is a front view of a sixth example of the tip according to the invention;
Figure 7a is a view of a detail of Figure 7a;
Figure 8 is a side view of a seventh embodiment of the tip according to the invention;
Figure 8a is a detail front view of the tip of Figure 8;
Figure 9 is a side view of an eighth embodiment of the tip according to the invention;
Figure 9a is a front detail view of the tip of Figure 9;
Figure 10 is a side view of a ninth embodiment of the tip according to the invention;
Figure 10a is a plan view of the tip of Figure 10;
Figure 10b is a front view of the tip of Figure 10;
Figure 11 is a front view of a tenth embodiment of the tip according to the invention;
Figure 11a is a side view of the tip of Figure 11;
Figure 12 is a side view of an eleventh embodiment of the tip according to the invention;
Figure 12a is a front view of the tip of Figure 12.

In the embodiments that follow, individual characteristics, given in relation to specific examples, may actually be interchanged with other different characteristics that exist in other embodiments.

Moreover, it is noted that anything found to be already known during the patenting process is understood not to be claimed and to be the subject of a disclaimer.

With reference to the figures, the reference numeral 1 generally designates a tip according to the invention, in a first embodiment, associated with an actuation handpiece, generally designated by the reference numeral 2.

The tip according to the present invention has been conceived and optimized in particular for dental bone microsurgery, and therefore for example for actions such as lifting the maxillary sinus, the separation of edentulous crests, simple and complex extractions, the inclusion of dental elements, periodontal resection surgery, cyst excision and others. However, it is noted that the tip according to the present invention can be used effectively also for maxillofacial, otorhinolaryngologic, neurological, orthopedic and other types of surgery without any kind of limitation.

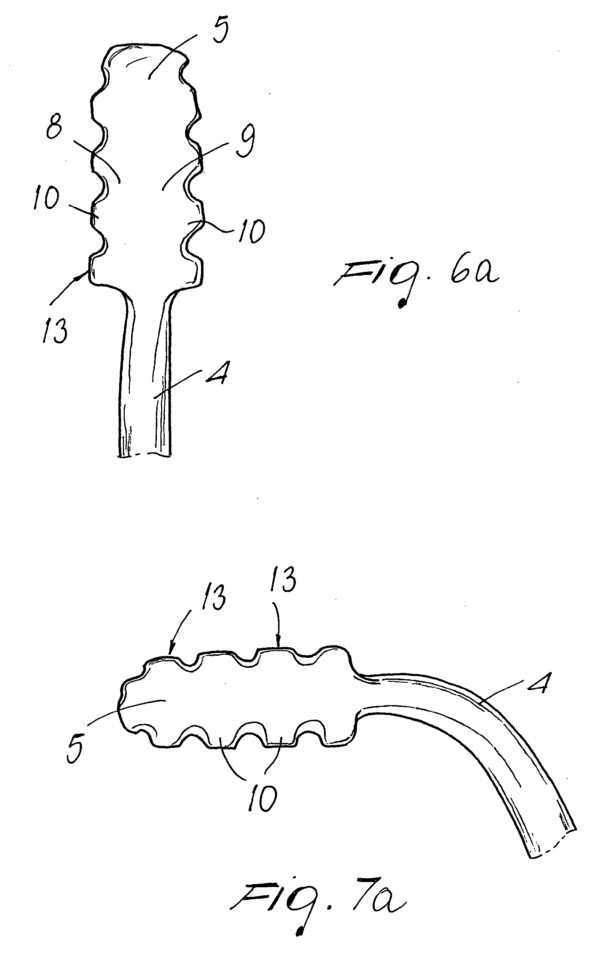

With reference to Figure 2, the tip for microsurgery comprises at least one coupling 3, which is suitable for connection to an actuation handpiece 2, which is extended by at least one narrow and substantially S-shaped stem 4, which in turn ends with at least one head 5 suitable to produce cuts and/or incisions in the bone portion involved in the procedure. The coupling 3 has an expanded portion 6, which has a substantially hexagonal transverse cross-section suitable for grip by a key or other actuation tool, and ends with a threaded shank 7 for detachable connection to the handpiece 2.

According to the invention, the head 5 advantageously comprises, along at least one of the lateral sides 8, 9, a distribution of sharp teeth 10, which have a preset thickness, height and width.

With reference now to Figure 2a, which is a front detail view of Figure 2, it can be seen that conveniently each one of the lateral sides 8, 9 of the head 5 is provided with a respective distribution of sharp teeth 10, so as to give symmetry in geometry and application to the tip 1.

Conveniently, the distributions of sharp teeth 10 are provided so that they are substantially uniform, i.e., there is a spacing of substantially constant length between one sharp tooth 10 and the immediately adjacent one.

As can be seen in Figure 2a, the front 11 of the head 4 also is affected by an additional distribution of front sharp teeth 12, which give greater operating versatility to the tip.

The sharp teeth 10 and the front sharp teeth 12 can be of course provided in any number.

The head 5 has a substantially flat configuration and its thickness is preferably approximately 0.2 mm; it is arranged, by way of non-limiting example, substantially at right angles to the imaginary longitudinal plane of symmetry of the actuation handpiece 2. However, it is noted that the inclination of the head with respect to such ideal plane may actually be any according to the specific requirements of use.

Each one of the sharp teeth 10 has a substantially straight terminal edge 13, which can be provided alternatively with other geometries dictated by the specifications of the application.

The tip 1 according to the invention is preferably made of natural steel. As an alternative, the tip can be made effectively of steel treated with titanium nitride, diamond steel, or also nitrided titanium alloy, so as to give greater hardness and extend the life of said tip.

The tip can be affected by an irrigation channel suitable to cool it.

It has been found both theoretically and experimentally that the tip according to the invention is particularly suitable for use in association with a handpiece 2 of the type with pneumatic-mechanical actuation.

Such handpiece is in fact designed and manufactured so as to give the tip an oscillating motion characterized by a substantially elliptical path having a preset breadth and frequency. This, together with the presence of sharp teeth 10 along the lateral sides 8, 9 of the head 5, allows the surgeon to perform cuts and incisions of different length and depth very simply and rapidly, even in difficult positions and in limited viewing conditions.

The pneumatically-actuated handpiece is suitable to perform an oscillating motion at the frequency of approximately 6000 Hz: this relatively low frequency limits considerably the generation of heat in cutting and therefore has more limited cooling requirements and causes substantially modest discomfort to the patient.

The method of use of the tip according to the invention, associated with a pneumatic-mechanical actuation handpiece, is directly derivable from what has been described.

It has thus been shown that the invention achieves the proposed aim and objects. The ability of the tip to cut and provide incisions on the lateral sides 8, 9 allows to provide in the bone extremely narrow, precise and deep cuts, in awkward and scarcely visible positions and with extremely limited inconvenience for the patient.

One should not underestimate the aspect of the operating safety of the tip, which can be controlled simply even by less expert operators, since it is substantially incapable of damaging soft tissues such as vessels, nerves, membranes, mucous membranes, etc. If the tip is subjected to excessive pressure, it tends to stop, indeed by virtue of the functional characteristics of the handpiece, and therefore the degree of safety is raised to a level that cannot be reached by piezoelectric handpieces.

The extremely limited thickness of the head 5 and the low frequency at which it operates increase the flexibility and maneuverability of the tip and limit the generation of heat; moreover, the overall cost of the apparatus is also rather limited, since the tip can be used on pneumatic handpieces already in use for other applications in many dental surgeries, which are therefore already commercially available.

The invention thus conceived is susceptible of numerous modifications and variations, all of which are within the scope of the inventive concept.

A second embodiment of the tip according to the invention is illustrated in Figures 3, 3a. In this embodiment, the head 5 is advantageously arranged substantially parallel to the imaginary longitudinal plane of symmetry of the handpiece, and the edges 13 of the sharp teeth 10 and of the sharp teeth 12 are substantially rounded, so as to obtain cuts and incisions having a different geometry.

A third embodiment of the tip according to the invention is shown in Figures 4, 4a. The head 5 here is oriented substantially at right angles to the longitudinal plane of symmetry of the handpiece and has a smaller number of sharp teeth 10; therefore, the respective edges 13 are longer.

Figures 5, 5a relate to a fourth embodiment of the tip according to the invention. The head 5 is arranged substantially parallel to the longitudinal plane of symmetry of the handpiece and has a large number of sharp teeth 10 for each one of the lateral sides 8, 9, each having a substantially straight edge 13.

Figures 6, 6a illustrate a fifth embodiment of the tip according to the invention. The head 5 here is provided substantially at right angles to the longitudinal plane of symmetry of the handpiece and has no front sharp teeth. Therefore, it is particularly suitable for cutting operations to be fought to be performed exclusively along the lateral sides 8, 9.

A sixth embodiment of the tip according to the invention is illustrated in Figures 7, 7a. Here the tip has a stem 4 which is folded in an S-shape with short radii of curvature, so as to reach easily bone parts located in regions affording limited viewing and limited space for operation.

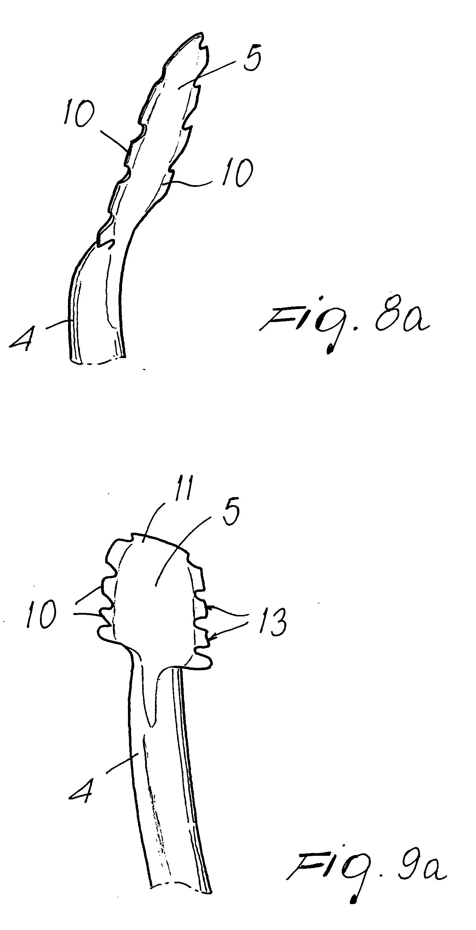

Figures 8, 8a and 9, 9a are related respectively to a seventh and an eighth embodiment of the tip according to the invention. In these examples, the head 5 is conveniently oriented with a preset angle with respect to the longitudinal plane of symmetry of the handpiece: this configuration provided further advantages in operation in relation to the execution of cuts and incisions in regions that are covered and difficult to reach.

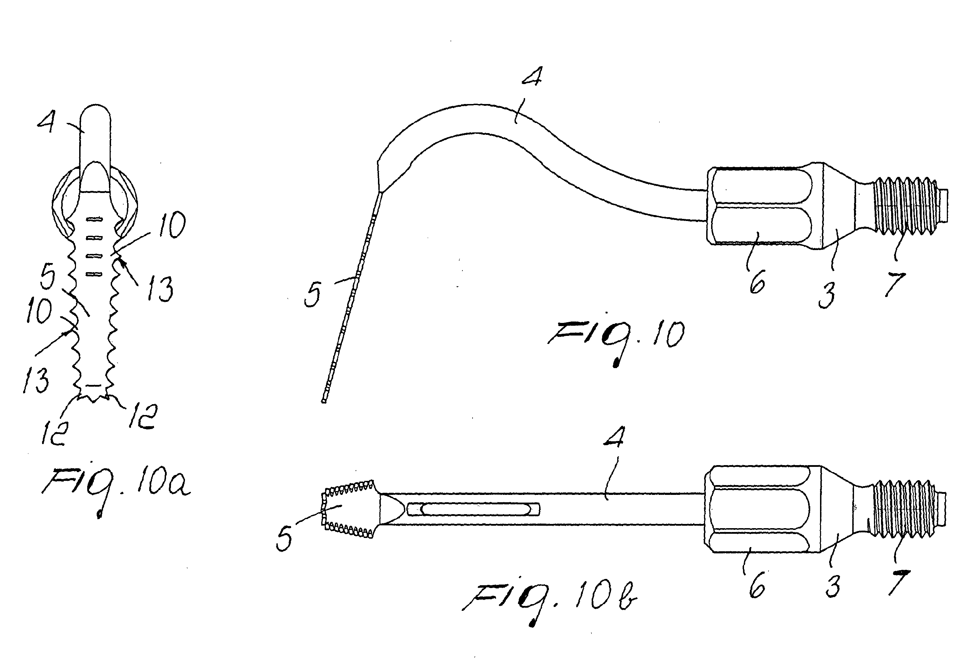

A ninth embodiment of the tip according to the invention is shown in Figures 10, 10a, 10b. The cutting head 5 here is oriented substantially at right angles to the longitudinal plane of symmetry of the handpiece and has sharp teeth 10 and front sharp teeth 12 provided with substantially pointed edges 13; accordingly, completely different cutting conditions are provided with respect to the conditions that occur when using a head with teeth having straight or rounded edges.

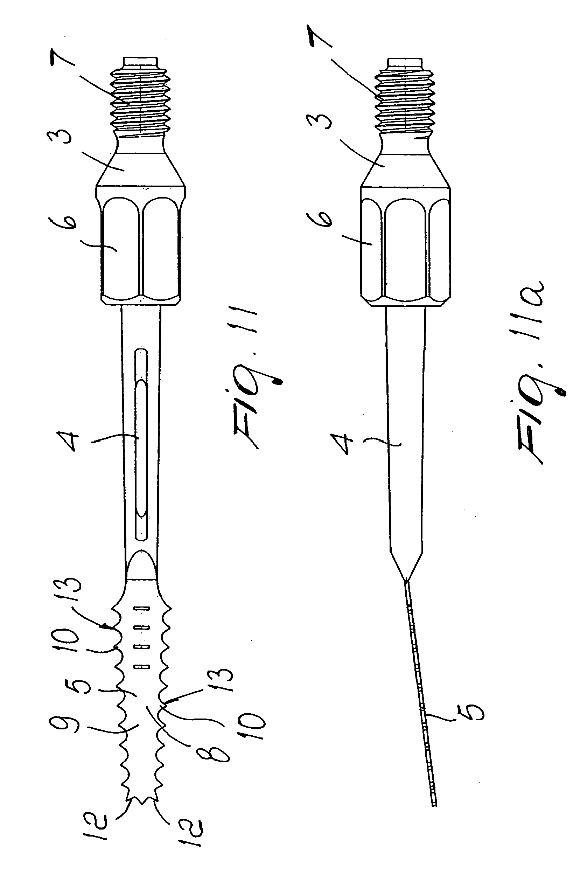

Figures 11, 11a relate to a tenth embodiment of the tip according to the invention, which is provided with a substantially straight stem 4; this allows conveniently to position the tip with respect to the bone with different inclinations with respect to the ones that can be obtained with a substantially S-shaped stem.

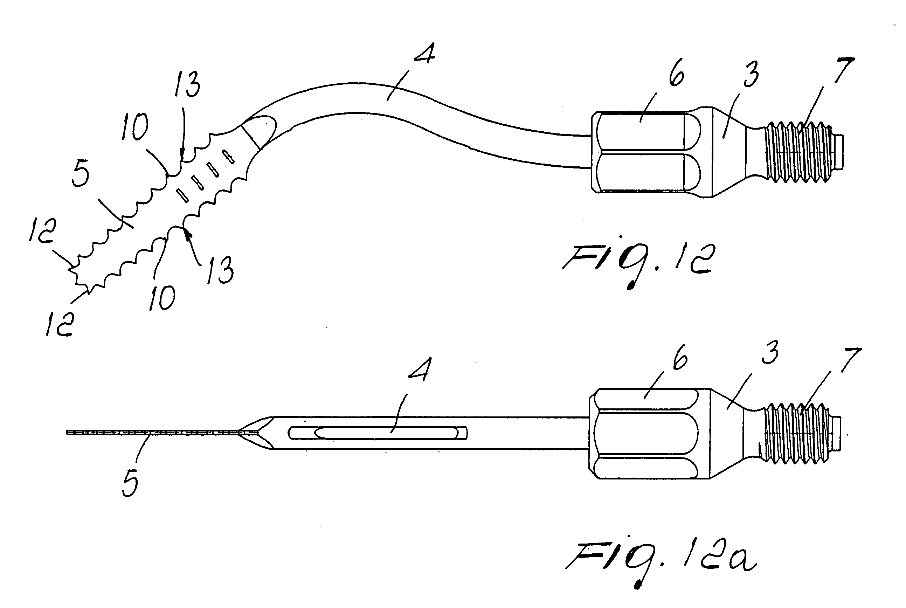

Figures 12, 12a relates to an eleventh embodiment of the tip according to the invention, in which the head 5 is arranged substantially parallel to the longitudinal plane of symmetry of the handpiece and the edges 10 of the sharp teeth 10 and of the front sharp teeth 12 are substantially pointed.

All the details may furthermore be replaced with other technically equivalent ones.

In practice, the materials used, as well as the shapes and the dimensions, may be any according to the requirements without thereby abandoning the scope of the protection of the claims that follow.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A tip for bone microsurgery, of the type that comprises at least one coupling (3), which is adapted for connection to an actuation handpiece (2), which is extended by at least one stem (4) ending with at least one head (5) adapted to produce cuts and/or incisions, which can be oriented in all the directions of space, in the portion of bone involved, **characterized in that** said head (5) has, along at least one of the lateral sides (8, 9), a distribution of sharp teeth (10) having a preset thickness, height and width.

2. The tip according to claim 1, **characterized in that** each one of said lateral sides (8, 9) of said head (5) is provided with a respective distribution of sharp teeth (10).

3. The tip according to claims 1 and 2, **characterized in that** said distributions of sharp teeth (10) are substantially uniform.

4. The tip according to one or more of the preceding claims, **characterized in that** the front (11) of said head (5) is affected by an additional distribution of front sharp teeth (12).

5. The tip according to one or more of the preceding claims, **characterized in that** each one of said sharp teeth (10) has a substantially straight terminal edge (13).

6. The tip according to one or more of claims 1 to 4, **characterized in that** each one of said sharp teeth (10) has a substantially pointed terminal edge (13).

7. The tip according to one or more of claims 1 to 4, **characterized in that** each one of said sharp teeth (10) has a substantially rounded terminal edge (13).

8. The tip according to one or more of the preceding claims, **characterized in that** said stem (4) is substantially straight.

9. The tip according to one or more of the preceding claims, **characterized in that** said stem (4) is substantially curved in an S-shape.

10. The tip according to one or more of the preceding claims,
**characterized in that** said head (5) is substantially flat and has a preset thickness.

11. The tip according to one or more of the preceding claims, **characterized in that** said head (5) is substantially parallel with respect to the longitudinal plane of symmetry of said handpiece (2).

12. The tip according to one or more of the preceding claims, **characterized in that** said head (5) is arranged substantially at right angles to the longitudinal plane of symmetry of said handpiece (2).

13. The tip according to one or more of the preceding claims, **characterized in that** said head (5) is arranged so as to form a preset angle with respect to the longitudinal plane of symmetry of said handpiece (2).

14. The tip according to one or more of the preceding claims, **characterized in that** it is affected by at least one irrigation channel suitable for cooling.

15. The tip according to one or more of the preceding claims, **characterized in that** it is made of steel.

16. The tip according to one or more of the preceding claims, **characterized in that** it is made of steel treated with titanium nitride.

17. The tip according to one or more of the preceding claims, **characterized in that** it is made of diamond steel.

18. The tip according to one or more of the preceding claims, **characterized in that** it is made of nitrided Ni-Ti alloy.

19. The tip according to one or more of the preceding claims, **characterized in that** said head (5) has a thickness of 0.2 mm.

20. A device for bone microsurgery, **characterized in that** it is constituted by at least one pneumatically-mechanically actuated handpiece (2), to which a tip (5) is rigidly connected at one end, said tip comprising at least one coupling (3), which is suitable for connection to said handpiece (2), which is extended by at least one stem (4) which ends with at least one head (5) adapted to produce cuts and/or incisions, which can be oriented in all the dimensions of space, in the involved bone portion, said head (5) having, along at least one of the lateral sides (8, 9), a distribution of sharp teeth (10) having a preset thickness, height and width.

21. The device according to claim 20, **characterized in that** said handpiece (2) is adapted to give said tip an oscillating motion which has a substantially elliptical path.

22. The device according to claim 20 and 21, **characterized in that** said handpiece (2) is adapted to perform an oscillating motion at the frequency of approximately 6000 Hz.
